# EUROPÄISCHE PATENTSCHRIFT

(11) **EP 1 641 569 B1**
(45) Veröffentlichungstag und Bekanntmachung des Hinweises auf die Patenterteilung: **27.10.2010**
(21) Anmeldenummer: 04737131.5
(22) Anmeldetag: 23.06.2004
(51) Int. Cl.: B05B 11/00, A61M 15/00

(54) **MIKROSTRUKTURIERTE HOCHDRUCKDÜSE MIT EINGEBAUTER FILTERFUNKTION**
MICROSTRUCTURED HIGH PRESSURE NOZZLE WITH AN IN-BUILT FILTER FUNCTION
AJUTAGE HAUTE PRESSION MICROSTRUCTURE COMPORTANT UNE FONCTION DE FILTRAGE INTEGREE

(30) Priorität: 30.06.2003 DE 10330370; 04.12.2003 EP 03027927
(43) Veröffentlichungstag der Anmeldung: 05.04.2006
(73) Patentinhaber: Boehringer Ingelheim International GmbH, 55216 Ingelheim am Rhein (DE)
(72) Erfinder: EICHER, Joachim, 44227 Dortmund (DE); GESER, Johannes, 55218 Ingelheim (DE); HAUSMANN, Matthias, 44287 Dortmund (DE); REINECKE, Holger, 44229 Dortmund (DE)
(74) Vertreter: Hammann, Heinz
(86) Internationale Anmeldenummer: PCT/EP2004/006768
(87) Internationale Veröffentlichungsnummer: WO 2005/000476

(56) Entgegenhaltungen:
- EP-B- 1 017 469
- US-A- 5 997 263
- US-B1- 6 503 362

## Beschreibung

Die vorliegende Erfindung betrifft eine mikrostruktürierte Hochdruckdüse mit eingebauter Filterfunktion für einen Hochdruckzerstäuber zum Vernebeln von medizinischen Flüssigkeiten.

### Stand der Technik .

Der Inhalationstherapie kommt eine immer stärkere Bedeutung bei der Therapie von Atemwegserkrankungen wie Asthma oder COPD zu.

Seit Bannung der Fluorchlorkohlenwasserstoff-betriebenen Treibgas-formulierungen wird mit zunehmenden Erfolg an vergleichbar effektiven oder verbesserten Ansätzen zur Erzeugung von lungengängigen Aerosolen gearbeitet.

Mit den internationalen Patentanmeldungen WO 91/14468 und WO 97/12687 wurde ein neuer Ansatz für Inhalatoren vorgestellt, die sich nicht allein dadurch auszeichnen, dass sie ein treibmittelfreies Aerosol auf gut verträglicher wässriger Basis bereitstellen, dessen Tröpfchenverteilung bezüglich der Aufnahme in der Lunge maßgeschneidert ist, sondern die zudem eine handliche Größe besitzen, die vergleichbar der Größe der bekannten treibmittelbetriebenen Inhalatoren ist.

Dieser auch als Respimat^{®} bezeichneter Vernebler kann flüssige Arzneimittellösungen in einer Menge von bevorzugt weniger als 20 Mikroliter mittels eines einzigen Hubs in ein Aerosol mit einer durchschnittlichen Teilchengröße von weniger als 10 Mikrometern zerstäuben. Dadurch kann die therapeutisch wirksamen Dosis des Arzneimittels mittels kleinster Volumina dem Patienten verabreicht werden.

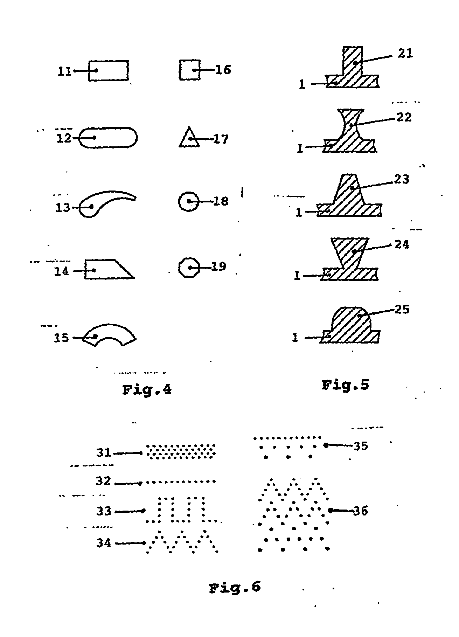

Bei diesem Vernebler wird eine Arzneimittellösung zunächst aus einem Reservoir über eine Kanüle mit einem integrierten Ventilkörper in eine Druckkammer gepumpt und von dort mittels hohen Drucks von bis zu 500 bar durch eine Düse in ein lungengängiges Aerosol überführt und versprüht. Dabei wird der Druck über eine Schraubenfeder erzeugt, die vor jedem Hub vom Patienten unter geringem Kraftaufwand erneut gespannt wird. Simultan mit der Spannbewegung wird die Druckkammer mit der Arzneimittellösung befüllt. Einzelheiten zu diesem Mechanismus können den Figuren 6a und 6b der WO 97/12687 entnommen werden.

Im wesentlichen besteht dieser Zerstäuber aus
- einem Gehäuseoberteil,
- einer Düse in dem Gehäuseoberteil,
- einem Federgehäuse, das mit dem Gehäuseoberteil verbunden ist und auf das das Gehäuseunterteil aufgesetzt wird,
- einem Vorratsbehälter, der in einen durch das Federgehäuse definierten Innenraum eingesteckt werden kann und
- einem Hohlkolben mit integriertem Ventilkörper, wobei der Hohlkolben von dem Vorratsbehälter in Richtung Düse führt.

In dem Gehäuseoberteil befindet sich zudem einem Pumpengehäuse, an dessen einem Ende sich der Düsenkörper mit der Düse bzw. Düsenanordnung befindet. Der Hohlkolben mündet ebenfalls in das Pumpgehäuse. Zwischen ihm und der Düse befindet sich eine Druckkammer.

Das Federgehäuse ist drehbar mit dem Gehäuseoberteil verbunden und durch die Drehbewegung wird mittels eines Sperrspannwerks im Gehäuseoberteil die Feder letztlich gespannt.

Mit dem Spannen der Feder wird ein Abtriebsflansch bewegt, der sich im oberen Bereich des Federgehäuses befindet und an dem der Hohlkolben aufgehängt ist.

Der Hohlkolben mit Ventilkörper entspricht einer in der WO 97/12687 offenbarten Vorrichtungen.

Als Düse wird bevorzugt eine durch Mikrotechnik hergestellte Düse oder Düsenkörper verwendet. Eine solche mikrostrukturierte Düsenkörper wird beispielsweise in der WO-94/07607 oder der WO 99/16530 offenbart. Die gattungsbildende Düse der WO 99/16530 ist Ausgangspunkt für die vorliegende Erfindung. Daher wird auf die gesamte Schrift WO 99/16530, insbesondere die durch die EP 1017469 B1 beanspruchte Ausführungsform, mit allen Merkmalen Bezug genommen.

Der Düsenkörper besteht aus zwei fest miteinander verbundenen Platten, die bevorzugt aus Glas und/oder Silizium sind und von denen wenigstens eine Platte einen oder mehrere mikrostrukturierte Kanäle aufweist, die die Düseneinlassseite mit der Düsenauslassseite verbinden. Die Düsenauslassseite mit den Düsenöffnungen befinden sich bevorzugt auf der der Düseneinlassseite gegenüberliegenden Seite.

Die Düseneinlassseite einen Flüssigkeitseinlass oder mehrere Flüssigkeitseinlässe. Der Einlass oder die Einlässe kann/können als Vorfilter ausgebildet sein. Alternativ hierzu kann das Vorfilter gegenüber dem Einlass/den Einlässen in Strömungsrichtung auch separat nachgeschaltet sein.

Nach dem Passieren des Vorfilters strömt die Flüssigkeit durch einen Hauptfilter, der durch eine Vielzahl von Vorsprüngen gebildet wird.

In Strömungsrichtung gesehen hinter dem Hauptfilter befindet sich ein Filtrat-Sammelraum für bereits gefilterte Flüssigkeit.

Aus dem Sammelraum gelangt die Flüssigkeit zu einem Auslass, der bevorzugt in Form einer Düse mit einer oder mehreren Düsenöffnungen ausgebildet ist.

Das Hauptfilter weist eine Vielzahl von reihenweise bevorzugt zickzackförmig angeordneten Vorsprüngen auf, die aus einer - bevorzugt flachen - Grundplatte hervorragen und damit integraler Bestandteil der Grundplatte sind. Die Grundplatte wird durch eine - bevorzugt flache - Deckenplatte vollständig überdeckt. Dadurch wird eine Vielzahl von Kanälen zwischen den Vorsprüngen, der Grundplatte und der Deckplatte gebildet. Diese Kanäle bilden einen Durchgang von der Einlass-Seite zur Auslass-Seite der Filterdüse. Der Abstand zwischen der Grundplatte in der Umgebung der Vorsprünge und der Deckplatte innerhalb einer Reihe von Vorsprüngen ist etwa so groß ist wie die Breite der Kanäle auf der Seite der Vorsprünge, auf der die Flüssigkeit in die Reihe von Kanälen tritt. Durch einen oder mehrere längliche(n) Einlass-Schlitz(e) tritt ungefilterte Flüssigkeit in das Filter. Der/Die Einlasschlitz(e) sind etwa so hoch wie die aus der Grundplatte hervorragenden Vorsprünge auf der Einlass-Seite des Filters.

Die Grundplatte besteht bevorzugt aus Silizium. Diese Platte wird von oben bevorzugt von einer Glasplatte bedeckt.

Für die Herstellung der Düsen werden folgenden Schritte durchlaufen:
- Strukturieren einer Charge der Basisplatten;
- Verbinden der Charge der Basis- und Abdeckplatte miteinander; und
- Separieren der individuellen Düsenanordnungen.

Die Basisplatte wird vorzugsweise in einer per se bekannten Art und Weise durch Ätztechniken strukturiert. Die Höhen der oben beschriebenen Strukturen liegen zwischen 2 und 40 Mikrometer, gewöhnlich zwischen ungefähr 3 und 20 Mikrometer, vorzugsweise zwischen ungefähr 4 und 14 Mikrometer, und insbesondere zwischen 5 und 7 Mikrometer. Das Material, das für die Basisplatte verwendet wird, ist vorzugsweise ein monokristallines Silizium, da dieses billig ist und in einem Zustand verfügbar ist (d.h. in Wafern), in dem es ausreichend flach und parallel und von einer geringen Oberflächenrauhigkeit ist, und es kann mit der Abdeckplatte ohne die zusätzliche Aufbringung von Klebe mitteln oder anderen Materialien während des darauf folgenden Verbindungsvorgangs verbunden werden. Um eine Vielzahl von Düsenanordnungen in paralleler Weise herzustellen, wird eine Vielzahl von Strukturbasisplatten auf einem Wafer aus Silizium gebildet.

Nach dem Strukturieren wird die Siliziumplatte gereinigt. Die Siliziumplatte wird dann an einer Abdeckplatte durch anodisches Anbonden (vergleiche US-Patent 3,397,278 vom 13.8.1968, Pomerantz et al.) verbunden.

Als Abdeckplatte eignen sich z.B. Glaspaltten, wie Alkaliborsilikatglas, wie beispielsweise Pyrex, (#7740 Corning) oder Tempax (Schott). Diese können durch anodisches Bonden des Siliziums und des Glases verbunden werden.

Nach dem Bondprozeß wird die Komposit-Struktur in individuellen Einheiten (z B Quadrate) mittels einer sich schnell drehenden Diamantkreissäge unterteilt.

Mit diesem bekannten Filter ist das Ziel verfolgt worden, eine derartiges Düse für einen Inhalator der eingangs geschilderten Art (Respimat^{®}) wirtschaftlich herzustellen.

Überraschend hat sich nun gezeigt, dass die Düsen in ihrer Gesamtheit ein für die dauerhafte Anwendung vorteilhafteres und einheitlicheres Sprühbild zeigen, wenn die Konfiguration der Mikrostrukturen im Inneren der Düse verändert wird.

### Beschreibung der Erfindung

Vor diesem Hintergrund ist es die Aufgabe der vorliegenden Erfindung, das durchschnittliche Sprühbild über eine Vielzahl von Düsen zu verbessern.

Eine weitere Aufgabe besteht dabei darin, den Strömungswiderstand in der Düse nicht wesentlich zu erhöhen.

Eine weitere Aufgabe besteht darin, die erfindungsgemäße Düse in einen Inhalator vom Respimat^{®}-Typ einzusetzen.

Gelöst wird diese Aufgabe indem bei der gattungsgemäßen Düse in dem Bereich zwischen Filterstruktur und Düsenauslass, also dem Filtrat-Sammelraum, eine zweite Art von Mikrostrukturen ausgebildet und die sich von den Filterstrukturen unterscheidet. Im folgenden wird diese zweite Art von Mikrostrukturen Sekundärstruktur genannt und die Filterstrukturen werden als Primärstruktur zusammengefasst. In Strömungsrichtung ist diese Sekundärstruktur der Primärstruktur nachgeschaltet.

Erfindungsgemäß werden zum Ausbilden der Sekundärstruktur in dem Filtrat-Sammelraum zusätzliche Einbauelemente ausgebildet. Bevorzugt handelt es sich dabei um zylinderartige Erhebungen, die sich vom Boden der Grundplatte zu der Deckplatte erstrecken. Bevorzugt handelt es sich dabei um Zylinder mit rundem Querschnitt.

Vorteilhaft sind Einbauelemente deren Höhe der Höhe des Filtrat-Sammelraumes entsprechen.

Die Einbauelemente können aus der Grundplatte herausgearbeitet sein.

Bevorzugt sind dabei Ausbildungsformen, bei denen die Einbauelemente in jeweils parallelen Reihen der Anordnung ABAB mit bevorzugt äquidistanten Abständen innerhalb der Reihen A und B und zwischen den Reihen A und B angeordnet sind. Dabei sind die benachbarten Reihen A und B in Strömungsrichtung bevorzugt um den Durchmesser der Einbauelemente verschoben. Durch den Einsatz von Einbauelementen mit rundem Querschnitt entsteht dadurch eine Geometrie, bei der jedes der Einbauelemente das Zentrum eines gleichseitigen Sechsecks bildet, wobei jede Ecke durch eine Nachbar-Einbauelement gebildet wird (hexagonales Muster). Naturgemäß trifft dies auf die Einbauelemente an Randpositionen nur teilweise oder gar nicht zu.

Die Dimension der Einbauelemente wird so gewählt, dass sie zu keiner wesentlichen Erhöhung des Strömungswiderstandes führen. Dies wird dadurch erreicht, dass die Abstände zwischen den Einbauelementen, die jeweils einen Durchlasskanal für die durchströmende Flüssigkeit bilden, derart sind, dass die senkrecht zur Strömungsrichtung resultierende, effektiv für die Flüssigkeit durchlässige Querschnittsfläche größer ist als die entsprechende effektive Querschnittsfläche der Durchlasskanäle die durch die Filterstrukturen gebildet werden. Damit wird der das Strömungsverhalten der Flüssigkeit im Inneren der Düse am stärksten durch die Strukturen des Hauptfilters beeinflusst.

Der Querschnitt der Einbauelemente ist bevorzugt derart ausgebildet, dass der Strömungswiderstand für eine durchströmendes Flüssigkeit minimiert ist. Hierzu sind runde oder ovale Querschnitte bevorzugt.

Alternativ zu den vorstehend beschriebenen Querschnitten können diese auch dreieckig, trapezförmig oder viereckig sein, wobei die Ecken in Strömungsrichtung ausgerichtet sein sollten.

Vorteilhaft können Ausführungsformen mit Dimensionen und der Abständen der Einbauelemente zueinander sein, die unter Ausnutzung der Oberflächenspannung der Flüssigkeit Einfluss auf die Verdunstungsfähigkeit der Lösung nehmen.

Die Einbauelemente weisen besonders bevorzugt einen Abstand im Bereich von 0,005 mm bis 0,02 mm auf. Die Einbauelemente selbst haben gemäß einer bevorzugten Weiterbildung einen Durchmesser im Bereich von 0,005 mm bis 0,02 mm. Die Abstände sollen dabei größer sein, als die kleinsten Abstände der die zickzackförmige Filterstruktur aufbauenden Strukturen.

Die Dichte der Einbauelemente liegt bevorzugt bei 200000 bis 300000 pro Quadratzentimeter, stärker bevorzugt bei 250000 pro Quadratzentimeter.

Es hat sich jedoch auch als günstig erwiesen, die Einbauelemente mit einer konkaven oder, alternativ hierzu, einer konvexen Umfangswand auszubilden.

Die Strukturen des Hauptfilters sind vorteilhafterweise Vorsprünge, die sich in einer Zick-Zack-Konfiguration über die gesamte Breite des Innenraums der Düse erstrecken. Dabei zeigen die Spitzen dieser Konfiguration alternierend in Richtung Einlassseite und Auslasseite. Eine imaginäre, quer zur Hauptsrömungsrichtung liegende Mittellinie unterteilt die Konfiguration in zwei annähernd gleich große Bereiche.

Aufgrund der Zick-Zack-Anordnung der Bauelemente des Hauptfilters wird die Richtung der Flüssigkeit quasi rechtwinklig, von der ursprünglichen Strömungsrichtung aus betrachtet, verändert. Anschließend erfolgt in dem Sammelraum eine erneute Änderung der Strömungsrichtung, diesmal wieder zurück in die der ersten Drehrichtung entgegengesetzte Richtung in einem inneren Winkel von kleiner 90 Grad.

Die vorstehend genannten Vorsprünge können dabei nebeneinander liegend über die gesamte Breite des Filters angeordnet sein, um die zickzackförmige Konfiguration aufzubauen.

In einer ersten bevorzugten Ausführungsform sind die Einbauelemente auf der Seite des Auslasses in Strömungsrichtung hinter der Zick-Zack-Konfiguration ausgebildet. Dabei können sich die Einbauelemente von der imaginären Mittellinie der zickzackförmigen Konfiguration bis zu den Düsenöffnungen erstrecken.

Alternativ hierzu können in einer zweiten Ausführungsform die Einbauelemente bis in die in Richtung des Einlasses ragenden Spitzen des Filtersystems ausgebildet sein, wobei jedoch der Bereich vor der zickzackförmigen Konfiguration vorzugsweise ausgenommen ist.

In einer dritten Variante können die Einbauelemente in Strömungsrichtung sowohl vor als auch hinter der Zick-Zack-Konfiguration angeordnet sein.

In einer alternativen Ausführungsform können die Vorsprünge des Hauptfilters in mehreren Reihen kaskadenförmig angeordnet sein. Die näher an der Einlass-Seite des Filters angeordneten Vorsprünge können größer als die mehr an der Auslass-Seite des Filters angeordneten Vorsprünge sein.

Der Abstand zwischen der flachen Grundplatte und der flachen Deckplatte in der Umgebung jeder kaskadenförmig angeordneten Reihe von Vorsprüngen ist etwa so groß wie die Breite der Kanäle auf der Seite der Vorsprünge, auf der die Flüssigkeit in die Reihe von Kanälen eintritt. Dieser Abstand liegt zwischen der Hälfte und dem Doppelten der Kanalbreite. Dieser Abstand kann von Reihe zu Reihe - in Strömungsrichtung gesehen - abnehmen. Die Kanäle haben auf ihrer Eintrittsseite für der Flüssigkeit also einen annähernd quadratischen Querschnitt.

In allen Ausführungsformen kann der Abstand zwischen der flachen Grundplatte in der Umgebung der Vorsprünge und der flachen Deckplatte innerhalb einer Reihe von Vorsprüngen des Hauptfilters konstant sein. Im Bereich des Reihenendes, das in der Nähe der Auslass-Seite des Filter liegt, kann der Abstand größer sein als im Bereich des Reihenendes, das in der Nähe der Einlass-Seite des Filters liegt. Dieser Abstand kann von einem zum anderen Ende der Reihe von Vorsprüngen bevorzugt etwa linear zunehmen.

Die einander zugewandten Seiten zweier benachbarter Reihen von Vorsprüngen begrenzen einen zusammenhängenden Raum, in den die Flüssigkeit aus sämtlichen Kanälen zwischen den Vorsprüngen einer ersten Reihe strömt und aus dem die Flüssigkeit in sämtliche Kanäle zwischen den Vorsprüngen der benachbarten Reihe strömt. Vor der ersten Reihe von Vorsprüngen des Hauptfilters befindet sich ein Sammelraum mit länglichem Querschnitt, in den die ungefilterte oder grob gefilterte Flüssigkeit eingeleitet wird und aus dem die Flüssigkeit in sämtliche Kanäle zwischen den Vorsprüngen der ersten Reihe hineinströmt. Hinter der letzten Reihe von Vorsprüngen befindet sich der Filtrat-Sammelraum mit länglichem Querschnitt, in den die Flüssigkeit aus sämtlichen Kanälen der letzten Reihe hineinströmt und aus dem die gefilterte Flüssigkeit abgeleitet wird.

Die Vorsprünge des Hauptfilters können die Form von Stegen haben, die - in Strömungsrichtung gesehen - gerade oder gekrümmt sind. Ferner können die Vorsprünge die Form von - bevorzugt geraden - Säulen mit beliebigem Querschnitt haben, bevorzugt mit kreisrundem oder mehreckigen Querschnitt.

Die Länge der zwischen den Stegen verlaufenden Kanäle ist mindestens doppelt so groß wie ihre Höhe auf der Eintrittsseite der Flüssigkeit. Der Querschnitt der Kanäle ist annähernd quadratisch oder tonnenförmig oder trapezförmig; im letzteren Fall kann die längere Seite des Trapezes durch die Deckplatte gebildet werden. Die Kanäle haben zum Beispiel eine Länge von 5 bis 50 Mikrometer, eine Höhe von 2,5 bis 25 Mikrometer und eine Breite von 2,5 bis 25 Mikrometer. Die Breite der Kanäle kann zur Austrittsseite hin größer werden.

Der Abstand zwischen den Reihen von Vorsprüngen des Hauptfilters ist bevorzugt doppelt so groß wie die Kanalbreite auf der Eintrittsseite. Die Reihen von Vorsprüngen können parallel zueinander oder mäanderartig oder bevorzugt zickzackartig verlaufen. Die zickzackartig angeordneten Reihen können um einen Winkel von 2 bis 25 Grad gegeneinander geneigt sein.

Die auszufilternden Partikel werden durch die zickzackartig angeordneten Reihen von Vorsprüngen zuerst in den Bereichen auf der Einlass-Seite der Flüssigkeit abgeschieden, die in der Nähe der Auslass-Seite des Filters liegen, der Raum zwischen den Reihen von Vorsprüngen wächst - beginnend im Bereich der Auslass-Seite des Filters - allmählich zu. Das Filter ist erst annähernd vollständig verlegt und die Filterkapazität erschöpft, wenn der Einlassraum zwischen jeweils zwei Reihen von Vorsprüngen fast ganz mit auszufilternden Partikeln gefüllt ist.

Der Abscheidegrad des Filters ist relativ scharf definiert wegen der geringen Schwankung der Abmessungen der Kanäle. Das Filter benötigt keinen Zustromverteiler für die zu filtrierende Flüssigkeit und keinen Filtratsammler für die filtrierte Flüssigkeit.

Die gefilterte Flüssigekeit wird in dem Filtrat-Sammelraum einer Düse zugeleitet. Diese weist bevorzugt zwei aufeinander zu geneigte Öffnungen auf. Die Flüssigkeit wird dadurch durch die Düse in zwei Strahlen aufgeteilt, die aufeinander zu gerichtet sind, so dass sie sich hinter der Düsenöffnung treffen.

Bei bevorzugten Ausführungsformen sind im Inneren der Düse in Strömungsrichtung zunächst die primäre Filterstruktur und anschließend die Sekundärstruktur ausgebildet. Dabei erstreckt sich die Filterstruktur über die gesamte Breite des im Inneren der Düse gebildeten Hohlraums und über eine Länge von bevorzugt 30 bis 70%, stärker bevorzugt 40 bis 50% der Gesamtlänge des im Inneren der Düse gebildeten Hohlraums. Bevorzugt beginnen die Filterstrukturen unmittelbar nach oder mit dem Düseneinlass. In besonders bevorzugten Ausführungsformen weist der Filterbereich zwei Arten von Filtersystemen auf: Einen vorgeschalteten Grobfilter und einen feinen Hauptfilter. Der Grobfilter kann aus einer einzigen Reihe von Strukturelementen aufgebaut sein, die parallel über die Raumbreite ausgebildet ist. Der Hauptfilter weist bevorzugt die bereits angesprochene Zick-Zack-Konfiguration auf. Die Sekundärstruktur ist dann in dem Bereich zwischen Filterende und Düsenauslass ausgebildet.

Die erfindungsgemäße Düse kann nach gemäß dem vorstehend angesprochenen Verfahren aus Metall, Silizium, Glas, Keramik oder Kunststoff hergestellt werden. Für die Grundplatte kann dasselbe oder ein anderes Material als für die Deckplatte verwendet werden. Das Filter ist für den Hochdruckbereich, z. B. bis 30 MPa (300 bar), geeignet.

Bei der Herstellung der erfindungsgemäßen Düse wird in Abweichung von dem aus dem Stand der Technik bekannte Verfahren der mit der Glasplatte fest verbundene, mikrostrukurierte Siliziumwafer bodenseitig mit einer adhäsiven Folie versehen, bevor die einzelnen Düsen aus der Platte herausgearbeitet werden.

Von besonderer Bedeutung ist die erfindungsgemäße mikrostrukturierte Filterdüse zum Filtrieren und Zerstäuben eines in einem Lösemittel gelösten Arzneimittels zum Erzeugen eines Aerosols für die inhalative Applikation. Geeignete Lösungsmittel sind beispielsweise Wasser oder Ethanol oder Mischungen davon. Geeignete Arzneimittel sind beispielsweise Berotec (Fenoterolhydrobromid), Atrovent (Ipratropiumbromid), Berodual (Ipratropiumbromid plus Fenoterolhydrobromid), Salbutamol (als Sulfat oder freie Base), Combivent (Ipratropiumbromid plus Salbutamol), Oxivent, Tiotropiumbromid und andere.

Die vorliegende Erfindung umfasst damit nicht nur die oben beschriebenen erfindungsgemäßen Düsen, sondern auch deren großtechnisches Herstellverfahren, die dadurch erhältlichen Düsen, sowie Inhalatoren, bevorzugt solche des Respimat^{®}-Typs, die diese Düse enthalten und mit denen bevorzugt medizinisch wirksam Inhalationsformulierungen zerstäubt werden können.

Die erfindungsgemäße mikrostrukturierte Filterdüse hat neben den bereits erwähnten auch noch folgende Vorteile:
- Wegen der Vielzahl von Kanälen auf kleiner Fläche bleibt sie funktionsfähig, auch wenn einige Kanäle durch Verunreinigungen der Flüssigkeit verstopft werden. Diese Eigenschaft ist entscheidend für die Brauchbarkeit des Filters, das mit einer Düse zusammengebaut ist. Bei Verwendung in einem Zerstäuber zur Verabreichung eines Medikamentes kann ein Versagen des Zerstäubers innerhalb einer angegebenen Gebrauchsdauer gravierende Folgen für den Anwender haben.

- Die Kanäle sind nach Form, Querschnittsfläche und Länge innerhalb enger Grenzen definiert. Im Spezialfall sind die Abmessungen aller Kanäle innerhalb eines Filters gleich.
- Der Kanalquerschnitt kann an weitere Bedingungen, zum Beispiel an den Querschnitt einer nachgeschalteten Düse, angepasst werden.
- Innerhalb eines kleinen Filtervolumens lässt sich eine große Filterfläche unterbringen.
- Die Strömung der Flüssigkeit ist vor ihrem Eintritt in die Kanäle zwischen den zickzackartig angeordneten Reihen im Wesentlichen senkrecht zur Strömung in den Kanälen gerichtet.
- Die offene Filterfläche (Summe der Querschnittsfläche aller Kanäle) beträgt mindestens 50 % der gesamten Filterfläche.
- Das Filter hat ein geringes Totvolumen, insbesondere bei einer hohen Dichte der Einbauelemente.
- Die Düsen können großtechnisch und in hoher Stückzahl produziert werden, bei geringem Ausschuß.
- Im Filtrat-Sammelraum werden Kristallisations- oder Präzipitationsprozesse in der Arzneimittelhaltigen Flüssigkeit aufgrund der Einbauelemente reduziert.
- Schließlich konnte durch die erfindungsgemäße Änderung des Aufbaus der Düse bei der großtechnischen Produktion der Anteil an Düsen, die kein dauerhaft einheitliches Sprühbild zeigen um ca. den Faktor 100 von 0,1-0,5% auf 0,001-0,005% verringert werden.

Die Erfindung wird nun anhand von Figuren näher erläutert.

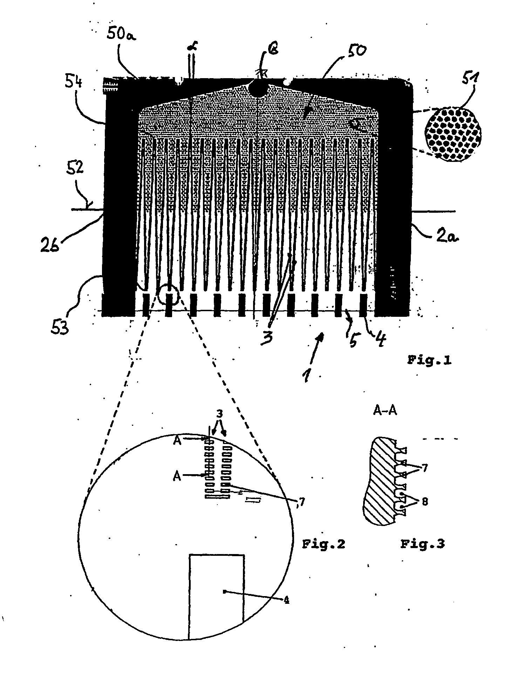

- Fig. 1: zeigt eine Ausführungsform der Filterdüse von der zunächst offenen Seite her gesehen, die anschließend mit der (nicht dargestellten) Deckplatte abgedeckt wird. Die Grundplatte (1) ist zwischen den Randbereichen (2a, 2b) mikrostrukturiert. Die Reihen (3) von Vorsprüngen sind zickzackartig angeordnet; die Reihen sind um den Winkel alpha gegeneinander geneigt. Auf der Eintrittsseite der Flüssigkeit befindet sich vor der Zickzackanordnung eine weitere Reihe von Vorsprüngen (4), die als Vorfilter dienen. Vor den Vorsprüngen (4) befinden sich Einlass-Schlitze (5), durch den die ungefilterte Flüssigkeit in das Filter eintritt. Bei dieser Ausführungsform ist im Anschluss an das Filter eine Düse (6) angeordnet, aus der die gefilterte Flüssigkeit austritt. Die Düse ist integraler Bestandteil der Grundplatte. Der zwischen den Filterreihen (3) und der Düse (6) gelegene Raum ist der Filtrat-Sammelraum. In diesem ist eine Sekundärstruktur (50) zwischen der Grundplatte und der Deckplatte angeordnet. Diese Sekundärstruktur umfasst eine große Anzahl von Einbauelementen (51), die sich zwischen diesen beiden Platten erstrecken. Die rechts dargestellte ausschnittsweise Vergrößerung veranschaulicht diesen Sachverhalt. Die Abstände zwischen den Einbauelementen (51) liegen bevorzugter Weise bei 0,02 mm von Mittelpunkt zu Mittelpunkt. Ebenso ist der Durchmesser der Einbauelemente (51) idealerweise 0,01 mm.
- Fig. 2: zeigt in vergrößerter Darstellung die Anordnung der Vorsprünge in den Reihen (3). Die Vorsprünge (7) sind in diesem Fall rechteckige Stege.
- Fig. 3: zeigt einen Querschnitt durch eine Reihe von Vorsprüngen entlang der Linie A-A in Fig. 2. Die Vorsprünge (7) haben konkav gekrümmte Längsseiten, zwischen denen Kanäle (8) mit tonnenförmigem Querschnitt liegen.
- Fig. 4: zeigt mehrere Ausführungsformen von Vorsprüngen, jeweils von der zunächst offenen Seite der Filterdüse her gesehen. Dargestellt sind ein rechteckiger Steg (11), ein länglicher Steg (12) mit konstanter Breite und gerundeten Schmalseiten, ein flügelartiger Steg (13), ein Steg (14) mit konstanter Breite und einer schräg verlaufenden Schmalseite und ein kreissegmentartig gebogener Steg (15). Weiter sind dargestellt: eine quadratische Säule (16), eine dreieckige Säule (17), eine runde Säule (18) und eine achteckige Säule (19).
- Fig. 5: zeigt Querschnitte durch verschiedene Stege, und zwar einen rechteckigen Querschnitt (21), einen Querschnitt (22) mit konkav gekrümmten Längsseiten, einen trapezförmigen Querschnitt (23), bei dem die lange Seite des Trapezes mit der Grundplatte (1) verbunden ist, einen trapezförmigen Querschnitt (24), bei dem die kurze Seite des Trapezes mit der Grundplatte (1) verbunden ist, und einen Steg (25) mit zwei abgerundeten Längskanten.
- Fig. 6: zeigt verschiedene Anordnungen von Vorsprüngen, wobei die Vorsprünge - unabhängig von ihrer Form - durch unterschiedlich große Punkte angedeutet sind. Die Vorsprünge können matrixförmig (31) angeordnet sein oder linear in einer Reihe (32) oder mäanderförmig (33) oder zickzackförmig (34). Mehrere reihenförmig (35), mäanderförmig oder zickzackförmig (36) angeordnete Vorsprünge können kaskadenartig hintereinander angeordnet sein.

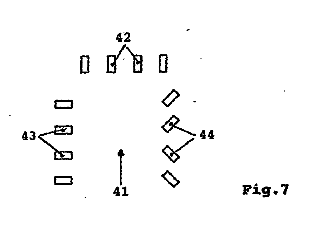

- Fig. 7: zeigt die Orientierung von Stegen in Bezug auf die Einströmungsrichtung (41) der Flüssigkeit. Die Stege (42) sind parallel zur Einströmungsrichtung angeordnet, die Stege (43) senkrecht zur Einströmungsrichtung und die Stege (44) unterschiedlich geneigt zur Einströmungsrichtung.

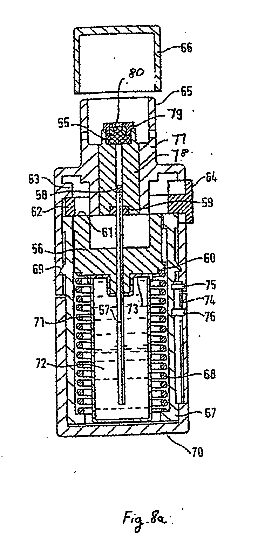

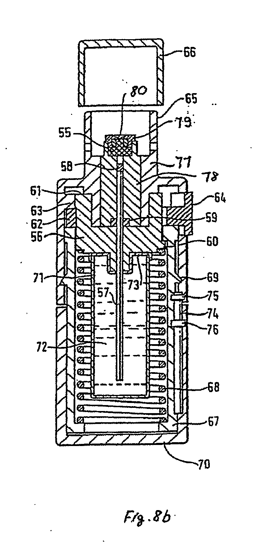

In den Figuren 8a/b, die identisch sind mit den Figuren 6 a/b der WO 97/12687, ist der Vernebler (Respimat®) beschrieben, mit dem die erfindungsgemäßen wässrigen Aerosolzubereitungen vorteilhaft inhaliert werden können.
Fig. 8a zeigt einen Längsschnitt durch den Zerstäuber bei gespannter Feder,
Fig. 8b zeigt einen Längsschnitt durch den Zerstäuber bei entspannter Feder.

Das Gehäuseoberteil (77) enthält das Pumpengehäuse (78), an dessen Ende der Halter (79) für die Zerstäuberdüse angebracht ist. In dem Halter befindet sich der erfindungsgemäße Düsenkörper (80) mit dem Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseoberteil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für die zu zerstäubende Flüssigkeit (72). Der Vorratsbehälter ist mit dem Stopfen (73) verschlossen, durch den der Hohlkolben in den Vorratsbehälter hineinragt und mit seinem Ende in die Flüssigkeit (Vorrat an Wirkstofflösung) eintaucht.

In der Mantelfläche des Federgehäuses ist eine Spindel (74) für das mechanische Zählwerk angebracht. An dem Ende der Spindel, das dem Gehäuseoberteil zugewandt ist, befindet ein Antriebsritzel (75). Auf der Spindel sitzt ein Reiter (76).

Ein Zerstäuber, mit dem aus einer medikamenthaltigen Flüssigkeit ein Aerosol erzeugt werden soll, enthält die erfindungsgemäße Düse, die ähnlich wie die in Fig. 1 dargestellte Düse aufgebaut ist.

Im folgenden wird eine bevorzugte Ausführungsform beschrieben. Die dabei angegebenen Zahlenwerte stellen inklusive Abweichungen von 20% bevorzugte Zahlenwerte dar.
Eine solche Düse hat eine Grundplatte von 2,6 mm Breite und etwa 5 mm Länge. Auf einer Breite von etwa 2 mm enthält sie bevorzugt 40 zickzackartig angeordnete Reihen von Vorsprüngen. Jede Reihe ist 1,3 mm lang. Die Vorsprünge sind rechteckige Stege, die 10 Mikrometer lang und 2,5 Mikrometer breit sind; sie ragen 5 Mikrometer aus der Grundplatte hervor. Zwischen den Stegen befinden sich Kanäle, die 5 Mikrometer hoch sind und 3 Mikrometer breit. Die Einbauelemente der Sekundärstruktur haben einen Durchmesser von 0,01 mm. Der Abstand der Einbauelemente beträgt ebenfalls 0,01 mm. Auf der Eintrittsseite der Flüssigkeit in die Düse befindet sich eine Reihe von 10 rechteckigen Stegen, die 200 Mikrometer lang und 50 Mikrometer breit sind; diese ragen 100 Mikrometer aus der Grundplatte hervor. Zwischen diesen Stegen befinden sich die Kanäle, die 100 Mikrometer hoch und 150 Mikrometer breit sind. Im Abstand von etwa 300 Mikrometer vor der Stegreihe befindet sich der Eintrittsspalt, der etwa 22 mm breit und 100 Mikrometer hoch ist.

Hinter den zickzackartig angeordneten Stegreihen befindet sich der Filtrat-Sammelraum, der 5 Mikrometer hoch ist und sich von 2 mm Breite allmählich verengt und in einer Düse mit rechteckigem Querschnitt mündet, der 5 Mikrometer hoch ist und 8 Mikrometer breit. Diese Düsenöffnung ist gleichzeitig mit der Mikrostrukturierung der Grundplatte erzeugt worden.

Ebenfalls ist in der Fig. 1 die Mittellinie 52 zu erkennen, welche die zickzackartige Anordnung der Reihen 3 in etwa halbem Abstand zwischen der eintrittsseitigen Spitze 53 und der austrittsseitigen Spitze 54 durchläuft.

### Bezugszeichenliste

- 1: Grundplatte
- 2a, 2b: Randbereich
- 3: Reihe von Vorsprüngen (7)
- 4: Vorsprünge (Vorfilter)
- 5: Einlass-Schlitz
- 6: Düse
- 7: Vorsprünge
- 8: Kanal
- 11, 12, 13,: Vorsprünge (7) in Form von Stegen
- 14,15 16,17,18,: Vorsprünge (7) in Form von Säulen
- 19 21: rechteckiger Stegquerschnitt
- 22: Stegquerschnitt mit konkaven Längsseiten
- 23: trapezförmiger Stegquerschnitt (lange Seite verbunden)
- 24: trapezförmiger Stegquerschnitt (kurze Seite verbunden)
- 25: Steg mit abgerundeten Längskanten
- 31: matrixartige Anordnung Vorsprünge (7)
- 32: lineare Anordnung Vorsprünge (7)
- 33: mäanderförmige Anordnung Vorsprünge (7)
- 34: zickzackförmige Anordnung Vorsprünge (7)
- 35: mehrere reihenförmige Anordnungen Vorsprünge (7)
- 36: Kaskadenartige Anordnung Vorsprünge (7)
- 41: Eintrittsöffnung Flüssigkeit
- 42: parallel zur Eintrittsöffnung ausgerichtete Stege
- 43: senkrecht zur Eintrittsöffnung ausgerichtete Stege
- 50: Sekundärstruktur
- 50a: Filtrat-Sammelraum
- 51: Einbauelemente
- 52: Mittellinie
- 53: Spitzen eintrittseitig
- 54: Spitzen austrittseitig
- 55: Filter
- 56: Abtriebsflansch
- 57: Hohlkolben
- 58: Ventilkörper
- 59: Dichtung
- 60: Anschlag
- 61: Anschlag
- 62: Sperrglied
- 63: Abstützung
- 64: Auslösetaste
- 65: Mundstück
- 66: Schutzkappe
- 67: Federgehäuse
- 68: Druckfeder
- 69: Schnappnasen
- 70: Gehäuseunterteil
- 71: Vorratsbehälter
- 72: Flüssigkeit
- 73: Stopfen
- 74: Spindel
- 75: Antriebsritzel
- 76: Reifen
- 77: Gehäuseoberteil
- 78: Pumpengehäuse
- 79: Halter
- 80: Düsenkörper

## Patentansprüche

1. Mikrostrukturierte Düse mit Filter, einem Einlass für unfiltrierte Flüssigkeit und einem Auslass für filtrierte Flüssigkeit, wobei die Düse umfasst:
eine im wesentlichen flache Grundplatte (1) und eine daran befestigbare Deckplatte;
einen als Primärstruktur ausgebildeten Hauptfilter mit mehreren in Reihen (3) nebeneinander angeordnete Vorsprünge (7), als integrale Komponenten der Grundplatte (1) und die jeweils davon abstehen, wobei die Vorsprünge (7) voneinander durch Kanäle (8) beabstandet sind, die einen Fluidweg durch die Düse von dem Einlass zu dem Auslass bilden, wobei die Deckplatte, wenn sie an der Grundplatte befestigt ist, die Vorsprünge (7) und die Kanäle (8) bedeckt; und
einen in Strömungsrichtung hinter dem Hauptfilter angeordneten Filtrat-Sammelraum (50a),
**dadurch gekennzeichnet,**
**dass** in dem Filtrat-Sammelraum (50a) eine Sekundärstruktur (50) ausgebildet ist, welche eine Vielzahl von an der Grundplatte (1) und/oder der Deckplatte angreifenden Einbauelementen (51) umfasst, die einen Durchmesser von 0,005 mm bis 0,02 mm aufweisen.

2. Mikrostrukturierte Düse mit Filter, einem Einlass für unfiltrierte Flüssigkeit und einem Auslass für filtrierte Flüssigkeit, wobei die Düse umfasst:
eine im wesentlichen flache Grundplatte (1) und eine daran befestigbare Deckplatte;
einer als Primärstruktur ausgebildeten Hauptfilter mit mehreren in Reihen (3) nebeneinander angeordnete Vorsprünge (7), als integrale Komponente der Grundplatte (1) und die jeweils davon abstehen und die in einer Zick-Zack-Konfiguration angeordnet sind, wobei die Vorsprünge (7) voneinander durch Kanäle (8) beabstandet sind, die einen Fluidweg durch die Düse von dem Einlass zu dem Auslass bilden, wobei die Deckplatte, wenn sie an der Grundplatte befestigt ist, die Vorsprünge (7) und die Kanäle (8) bedeckt;
und
einen in Strömungsrichtung hinter dem Hauptfilter angeordneten Filtrat-Sammelraum (50a),
**dadurch gekennzeichnet,**
**dass** indem Filtrat-Sammelraum (50a) eine Sekundärstruktur (50) ausgebildet ist, welche eine Vielzahl von an der Grundplatte (1) und/oder der Deckplatte angreifenden Einbauelementen (51) umfasst, die in Strömungsrichtung vor und locker hinter der Zick-Zack-Konfiguration ausgebildet sein können.

3. Mikrostrukturierte Düse nach Anspruch 2, **dadurch gekennzeichnet, dass** die Einbauelemente (51) einen Durchmesser von 0,005 mm bis 0,02 mm aufweisen.

4. Mikrostrukturierte Düse nach einem der Ansprüche 1 bis 3, **dadurch gekennzeichnet, dass** die Einbauelemente (51) eine zylindrische Umfangswand aufweisen.

5. Mikrostrukturierte Düse nach einem der Ansprüche 1 bis 4, **dadurch gekennzeichnet, dass** die Einbauelemente (51) untereinander einen Abstand von 0,005 mm bis 0,02 mm aufweisen.

6. Mikrostrukturierte Düse nach einem der Ansprüche 1 bis 5, **dadurch gekennzeichnet, dass** die Einbauelemente (51) einen Durchmesser von 0,01 mm aufweisen.

7. Mikrostrukturierte Düse nach einem der Ansprüche 1 bis 6, **dadurch gekennzeichnet, dass** die Einbauelemente (51) eine konkave Umfangswand aufweisen.

8. Mikrostrukturierte Düse nach einem der Ansprüche 1 bis 6, **dadurch gekennzeichnet, dass** die Einbauelemente (51) eine konvexe Umfangswand aufweise.

9. Mikrostrukturierte Düse nach einem der Ansprüche 1 bis 8; **dadurch gekennzeichnet, dass** die Einbauelemente sich pfeilerartig von der Grundplatte zur Deckplatte erstrecken und integraler Bestandteil der Deckplatte sind.

10. Mikrostrukturierte Düse nach einem der Ansprüche 1 bis 9, **dadurch gekennzeichnet, dass** die Vorsprünge (7) nebeneinander liegend über die gesamte Breite des Filters angeordnet sind.

11. Mikrostrukturierte Düse nach einem der Ansprüche 2 bis 10, **dadurch gekennzeichnet, dass** die Sekundärstruktur (50) auf der Seite des Auslasses der Zick-Zack-Konfiguration bis zu der Mittellinie (52) ausgebildet ist.

12. Mikrostrukturierte Düse nach einem der Ansprüche 2 bis 10, **dadurch gekennzeichnet, dass** die Sekundärstruktur (50) auf der Seite des Auslasses der Zick-Zack-Konfiguration bis in die in Richtung des Einlasses ragenden Spitzen ausgebildet ist.

13. Mikrostrukturierte Düse nach einem der Ansprüche 1 bis 12, **gekennzeichnet durch**
- einen Abstand zwischen der Grundplatte in der Umgebung der Vorsprünge und der Deckplatte innerhalb einer Reihe (3) von Vorsprüngen (7), der etwa so groß ist wie die Breite der Kanäle (8) auf der Seite der Vorsprünge (7), auf der die Flüssigkeit in die Reihe von Kanälen (8) eintritt.

14. Mikrostrukturierte Düse nach einem der Ansprüche 1 bis 13, g**ekennzeichnet durch**
- eine Vielzahl von in Reihen (3) nebeneinander angeordneten Vorsprüngen (7), die aus einer Grundplatte (1) hervorragen und die integraler Bestandteil der Grundplatte sind, wobei
- mehrere Reihen (3) von Vorsprüngen kaskadenförmig angeordnet sind und
- der Querschnitt der Kanäle (8) senkrecht zur Strömungsrichtung der Flüssigkeit - in Strömungsrichtung gesehen - von Reihe zu Reihe abnimmt und
- die näher an der Einlass-Seite des Filters angeordneten Vorsprünge (3) größer sind oder deren Anzahl höher ist, so dass deren Abstände kleiner werden als die mehr an der Auslass-Seite des Filters angeordneten Vorsprünge (3) und
- der Abstand zwischen der Grundplatte und der Deckplatte in der Umgebung jeder kaskadenförmig angeordneten Reihe von Vorsprüngen (7) etwa so groß ist wie die Breite der Kanäle auf der Seite der Vorsprünge (7), auf der die Flüssigkeit in die Reihe von Kanälen eintritt, und
- einen länglichen Einlass-Schlitz (5) für die ungefilterte Flüssigkeit, der sich über annähernd die gesamte Filterbreite erstreckt und der etwa so hoch ist wie die aus der Grundplatte hervorragenden Vorsprünge (7) auf der Einlass-Seite des Filters, und
- einen länglichen Auslass-Schlitz für die gefilterte Flüssigkeit, der sich über annähernd die gesamte Filterbreite erstreckt und der etwa so hoch ist wie die aus der Grundplatte hervorragenden Vorsprünge (7) auf der Auslass-Seite des Filters.

15. Mikrostrukturierte Düse nach den Ansprüchen 1 bis 14, **gekennzeichnet durch**
- eine flache Grundplatte (1) und eine flache Deckplatte.

16. Mikrostrukturierte Düse nach den Ansprüchen 1 bis 15, **dadurch gekennzeichnet; dass** alle Strukturen des Filters ausschließlich auf der Grundplatte (1) ausgebildet sind.

17. Mikrostrukturierte Düse nach den Ansprüchen 1 bis 16, **gekennzeichnet durch**
- einen Abstand zwischen der flachen Grundplatte (1) in der Umgebung der Vorsprünge (7) und der flachen Deckplatte innerhalb einer Reihe (3) von Vorsprüngen (7) zwischen der Hälfte und dem Doppelten der Kanalbreite auf der Seite der Vorsprünge, auf der die Flüssigkeit in die Reihe Kanälen (8) eintritt.

18. Mikrostrukturierte Düse nach den Ansprüchen 1 bis 17, **gekennzeichnet durch** mehrere Reihen von Vorsprüngen nebeneinander, wobei die einander zugewandten Seiten zweier benachbarter Reihen von Vorsprüngen (7) einen zusammenhängenden Raum begrenzen, in den die Flüssigkeit aus sämtlichen Kanälen zwischen den Vorsprüngen einer ersten Reihe strömt und aus dem die Flüssigkeit in sämtliche Kanäle zwischen den Vorsprüngen der in der Strömungsrichtung folgenden Reihe strömt.

19. Mikrostrukturierte Düse nach den Ansprüchen 1 bis 18, **dadurch gekennzeichnet, dass**
- der Sammelraum (50a) einen länglichen Querschnitt zwischen dem Einlass-Schlitz (5) und der ersten Reihe von Vorsprüngen (4) aufweist, in den die ungefilterte Flüssigkeit eingeleitet wird und aus dem die Flüssigkeit in sämtliche Kanäle zwischen den Vorsprüngen der ersten Reihe hineinströmt, und dass
- der Sammelraum (50a) einen länglichen Querschnitt zwischen der letzten Reihe von Vorsprüngen und dem Auslassschlitz aufweist, in den die Flüssigkeit aus sämtlichen Kanälen der letzten Reihe hineinströmt, und aus dem die gefilterte Flüssigkeit abgeleitet wird.

20. Mikrostrukturierte Düse nach den Ansprüchen 1 bis 19, **gekennzeichnet durch**
- Vorsprünge in Form von Stegen (11, 12, 13, 14, 15), die in Strömungsrichtung gesehen gerade oder gekrümmt sind, oder
- Vorsprünge in Form von Säulen (16, 17, 18, 19).

21. Mikrostrukturierte Düse nach den Ansprüchen 1 bis 20, **gekennzeichnet durch**
- Kanäle (8), deren Länge bei konstantem Querschnitt mindestens doppelt so groß ist wie ihre Höhe auf der Eintrittsseite der Flüssigkeit.

22. Mikrostrukturierte Düse nach den Ansprüchen 1 bis 21, **gekennzeichnet durch**
- zwischen Stegen verlaufende Kanäle (8) mit annähernd quadratischem Querschnitt, der über die Kanallänge konstant ist, mit einer Länge von 5 Mikrometer bis 50 Mikrometer, mit einer Höhe von 2,5 Mikrometer bis 25 Mikrometer und einer Breite von 2,5 bis 25 Mikrometer.

23. Mikrostrukturierte Düse nach den Ansprüchen 1 bis 22, **gekennzeichnet durch**
- Kanäle, deren Querschnitt tonnenförmig oder trapezförmig ist, wobei bevorzugt die längere Seite des Trapezes **durch** die Deckplatte gebildet wird.

24. Mikrostrukturierte Düse nach den Ansprüchen 1 bis 23, **gekennzeichnet durch**
- Kanäle (8) mit annähernd quadratischem Querschnitt auf der Eintrittsseite der Flüssigkeit und einem zur Austrittseite der Flüssigkeit breiter werdenden Querschnitt.

25. Mikrostrukturierte Düse nach den Ansprüchen 1 bis 24, **gekennzeichnet durch**
- einen Abstand zwischen den Reihen von Vorsprüngen, der bevorzugt doppelt so groß ist wie die Kanalbreite auf der Eintrittsseite.

26. Mikrostrukturierte Düse nach den Ansprüchen 1, 4 bis 10 oder 13 bis 25, **gekennzeichnet durch**
- Vorsprünge, die
- in parallel zueinander verlaufenden Reihen (31) oder
- in mäanderförmig verlaufenden Reihen (33) um einen Winkel alpha von 2 Grad bis 25 Grad gegeneinander geneigt sind.
angeordnet sind.

27. Mikrostrukturierte Düse nach den Ansprüchen 2 bis 25, **gekennzeichnet durch** Vorsprünge, die um einen Winkel alpha von 2 Grad bis 25 Grad gegeneinander geneigt sind.

28. Mikrostrukturierte Düse nach den Ansprüchen 1 bis 27, **gekennzeichnet durch**
- einen konstanten Abstand zwischen der flachen Grundplatte (1) in der Umgebung der Vorsprünge (7) und der flachen Deckplatte innerhalb einer Reihe (3) von Vorsprüngen.

29. Mikrostrukturierte Düse nach den Ansprüchen 1 bis 27, **gekennzeichnet durch** einen sich in Strömungsrichtung verjüngenden Abstand zwischen Grundplatte (1) und Deckplatte

30. Mikrostrukturierte Düse nach den Ansprüchen 1 bis 29, **gekennzeichnet durch**
- einen Abstand zwischen der flachen Grundplatte (1) in der Umgebung der Vorsprünge (7) und der flachen Deckplatte innerhalb einer Reihe von Vorsprüngen, der vom Bereich des Reihenendes, das in der Nähe der Einlass-Seite des Filters liegt, in Richtung zum Bereich des Reihenendes, das in der Nähe der Auslass-Seite des Filters liegt, zunimmt.

31. Mikrostrukturierte Düse nach den Ansprüchen 1 bis 30, **gekennzeichnet durch**
- eine Grundplatte (1), die **durch** isotropes oder anisotropes Nass- oder Trockenätzen oder eine Kombination dieser Verfahren strukturiert worden ist, bevorzugt **durch** anisotropes Trockenätzen.

32. Mikrostrukturierte Düse nach den Ansprüchen 1 bis 31, **gekennzeichnet durch**
- eine Grundplatte (1) aus Silizium und eine Deckplatte aus Glas, die **durch** anodisches Bonden gefügt ist.

33. Mikrostrukturierte Düse nach den Ansprüchen 1 bis 30, **dadurch gekennzeichnet, dass** der Filtrat-Sammelraum (50a) in Strömungsrichtung konisch zuläuft und als Auslass mindestens eine Düse (6) aufweist.

34. Mikrostrukturierte Düse nach den Ansprüchen 1 bis 31, **gekennzeichnet durch**
- eine Grundplatte (1) aus Silizium und eine Deckplatte aus Silizium, die **durch** direktes Bonden gefügt ist.

35. Mikrostrukturierte Düse nach den Ansprüchen 1 bis 34, **dadurch gekennzeichnet, dass** die Abstände zwischen den Einbauelementen, die jeweils einen Durchlasskanal für die durchströmende Flüssigkeit bilden, derart sind, dass die senkrecht zur Strömungsrichtung resultierende, effektiv für die Flüssigkeit durchlässige Querschnittsfläche größer ist als die entsprechende effektive Querschnittsfläche der Durchlasskanäle die durch die Strukturen des Hauptfilters gebildet werden.

36. Zerstäuber für die Inhalationstherapie, welcher eine mikrostrukturierte Düse nach einem vorangehenden Anspruch 1 bis 35 umfasst.

37. Verfahren zur Herstellung einer Düse nach einem der Ansprüche 1 bis 35, wobei in einem Schritt in eine Seite eines Siliziumwafers für eine große Anzahl von Düsen Mikrostrukturen in Form der Filterstrukturen, Sekundärstrukturen, Düseneinlass und Düsenauslass geätzt wird, in einem nachfolgenden Schritt auf diese Seite des Siliziumwafers eine Glasplatte fest fixiert wird, in einem davon unabhängigen Schritt der Siliziumwafer auf eine adhäsive Folie aufgebracht wird und in einem abschließenden Schritt aus der Baueinheit aus Siliziumwafer/Glasplatte mit der adhäsiven Folie auf der Unterseite des Siliziumwafers von der Seite der Glasplatte kommend mittels einer Diamantensäge die einzelnen Düsen herausgearbeitet werden.

## Claims

1. Microstructured nozzle having a filter, an inlet for unfiltered fluid and an outlet for filtered fluid, the nozzle comprising:
a substantially flat base plate (1) and a cover plate which may be attached thereto;
a main filter constructed as the primary structure, with a plurality of projections (7) arranged side by side in rows (3), as integral components of the base plate (1) and projecting therefrom, the projections (7) being spaced from one another by channels (8) which form a path for fluid through the nozzle from the inlet to the outlet, while the cover plate, if it is attached to the base plate, covers the projections (7) and the channels (8); and
a filtrate collecting chamber (50a) arranged behind the main filter in the direction of flow,
**characterised in that**
in the filtrate collecting chamber (50a) is disposed a secondary structure (50) which comprises a plurality of built-in elements (51) acting on the base plate (1) and/or the cover plate, having a diameter of 0.005 mm to 0.02 mm.

2. Microstructured nozzle having a filter, an inlet for unfiltered fluid and an outlet for filtered fluid, the nozzle comprising:
a substantially flat base plate (1) and a cover plate which may be attached thereto;
a main filter constructed as the primary structure, with a plurality of projections (7) arranged side by side in rows (3), as integral components of the base plate (1) and projecting therefrom and arranged in a zigzag configuration, the projections (7) being spaced from one another by channels (8) which form a path for fluid through the nozzle from the inlet to the outlet, while the cover plate, if it is attached to the base plate, covers the projections (7) and the channels (8); and
a filtrate collecting chamber (50a) arranged behind the main filter in the direction of flow,
**characterised in that**
in the filtrate collecting chamber (50a) is disposed a secondary structure (50) which comprises a plurality of built-in elements (51) acting on the base plate (1) and/or the cover plate, which may be formed in front of and loosely behind the zigzag configuration in the direction of flow.

3. Microstructured nozzle according to claim 2, **characterised in that** the built-in elements (51) have a diameter of from 0.005 mm to 0.02 mm.

4. Microstructured nozzle according to one of claims 1 to 3, **characterised in that** the built-in elements (51) have a cylindrical circumferential wall.

5. Microstructured nozzle according to one of claims 1 to 4, **characterised in that** the built-in elements (51) are at a spacing of 0.005 mm to 0.02 mm from one another.

6. Microstructured nozzle according to one of claims 1 to 5, **characterised in that** the built-in elements (51) have a diameter of 0.01 mm.

7. Microstructured nozzle according to one of claims 1 to 6, **characterised in that** the built-in elements (51) have a concave circumferential wall.

8. Microstructured nozzle according to one of claims 1 to 6, **characterised in that** the built-in elements (51) have a convex circumferential wall.

9. Microstructured nozzle according to one of claims 1 to 8, **characterised in that** the built-in elements extend in the manner of pillars from the base plate to the cover plate and are an integral part of the cover plate.

10. Microstructured nozzle according to one of claims 1 to 9, **characterised in that** the projections (7) are arranged side by side over the entire width of the filter.

11. Microstructured nozzle according to one of claims 2 to 10, **characterised in that** the secondary structure (50) is formed on the side of the outlet of the zigzag configuration up to the central line (52).

12. Microstructured nozzle according to one of claims 2 to 10, **characterised in that** the secondary structure (50) is formed on the side of the outlet of the zigzag configuration right into the points projecting in the direction of the inlet.

13. Microstructured nozzle according to one of claims 1 to 12, **characterised by**
- a spacing between the base plate in the area around the projections and the cover plate within a row (3) of projections (7), which is about the same size as the width of the channels (8) on the side of the projections (7) where the fluid enters the row of channels (8).

14. Microstructured nozzle according to one of claims 1 to 13, **characterised by**
- a plurality of projections (7) arranged side by side in rows (3), which project from a base plate (1) and are an integral part of the base plate, wherein
- several rows (3) of projections are arranged in a cascade and
- the cross section of the channels (8) decreases from row to row perpendicularly to the direction of flow of the fluid, viewed in the direction of flow, and
- the projections (3) arranged closer to the inlet side of the filter are larger or are more numerous, so that the spacings between them are smaller than the projections (3) arranged more on the outlet side of the filter and
- the spacing between the base plate and the cover plate in the area around each row of projections (7) arranged in a cascade is about the same size as the width of the channels on the side of the projections (7) where the fluid enters the row of channels, and
- an oblong inlet slot (5) for the unfiltered fluid which extends over approximately the entire width of the filter and is about the same height as the projections (7) protruding from the base plate on the inlet side of the filter, and
- an oblong outlet slot for the filtered fluid which extends over approximately the entire width of the filter and is about the same height as the projections (7) protruding from the base plate on the outlet side of the filter.

15. Microstructured nozzle according to claims 1 to 14, **characterised by**
- a flat base plate (1) and a flat cover plate.

16. Microstructured nozzle according to claims 1 to 15, **characterised in that** all the structures of the filter are formed exclusively on the base plate (1).

17. Microstructured nozzle according to claims 1 to 16, **characterised by**
- a spacing between the flat base plate (1) in the area around the projections (7) and the flat cover plate within a row (3) of projections (7) between half and twice the width of the channels on the side of the projections where the fluid enters the row of channels (8).

18. Microstructured nozzle according to claims 1 to 17, **characterised by**
- several rows of projections side by side, the facing sides of two adjacent rows of projections (7) defining a cohesive chamber into which the fluid from all the channels flows between the projections of a first row and out of which the fluid flows into all the channels between the projections of the next row in the direction of flow.

19. Microstructured nozzle according to claims 1 to 18, **characterised in that**
- the collecting chamber (50a) has an oblong cross section between the inlet slot (5) and the first row of projections (4) into which the unfiltered fluid is conveyed and out of which the fluid flows into all the channels between the projections of the first row, and **in that**
- the collecting chamber (50a) has an oblong cross section between the last row of projections and the outlet slot into which the fluid from all the channels of the last row flows, and out of which the filtered fluid is discharged.

20. Microstructured nozzle according to claims 1 to 19, **characterised by**
- projections in the form of posts (11, 12, 13, 14, 15) which are straight or curved, viewed in the direction of flow, or
- projections in the form of columns (16, 17, 18, 19).

21. Microstructured nozzle according to claims 1 to 20, **characterised by**
- channels (8), the length of which is at least twice their height at the entry side for the fluid, their cross section remaining constant.

22. Microstructured nozzle according to claims 1 to 21, **characterised by**
- channels (8) extending between posts, said channels being of approximately square cross section, which is constant over the length of the channel, with a length of 5 microns to 50 microns, with a height of 2.5 microns to 25 microns and a width of 2.5 to 25 microns.

23. Microstructured nozzle according to claims 1 to 22, **characterised by**
- channels whose cross section is barrel-shaped or trapezoidal, the longer side of the trapezium preferably being formed by the cover plate.

24. Microstructured nozzle according to claims 1 to 23, **characterised by**
- channels (8) of approximately square cross section on the fluid inlet side and a cross section which becomes wider towards the fluid outlet side.

25. Microstructured nozzle according to claims 1 to 24, **characterised by**
- a spacing between the rows of projections which is preferably twice the size of the channel width on the inlet side.

26. Microstructured nozzle according to claims 1, 4 to 10 or 13 to 25, **characterised by**
- projections which are arranged
- in rows (31) running parallel to one another or
- in rows (33) in a meandering pattern which are inclined towards one another at an angle alpha of 2 degrees to 25 degrees.

27. Microstructured nozzle according to claims 2 to 25, **characterised by** projections which are inclined towards one another at an angle alpha of 2 degrees to 25 degrees.

28. Microstructured nozzle according to claims 1 to 27, **characterised by**
- a constant spacing between the flat base plate (1) in the area around the projections (7) and the flat cover plate within a row (3) of projections.

29. Microstructured nozzle according to claims 1 to 27, **characterised by** a spacing between the base plate (1) and cover plate which tapers in the direction of flow.

30. Microstructured nozzle according to claims 1 to 29, **characterised by**
- a spacing between the flat base plate (1) in the area around the projections (7) and the flat cover plate within a row of projections, which increases from the region of the end of the row located close to the inlet side of the filter, towards the region of the end of the row located close to the outlet side of the filter.

31. Microstructured nozzle according to claims 1 to 30, **characterised by**
- a base plate (1) which has been structured by isotropic or anisotropic wet or dry etching or a combination of these methods, preferably by anisotropic dry etching.

32. Microstructured nozzle according to claims 1 to 31, **characterised by**
- a base plate (1) made of silicon and a cover plate made of glass, attached by anodic bonding.

33. Microstructured nozzle according to claims 1 to 30, **characterised in that** the filtrate collecting chamber (50a) tapers conically in the direction of flow and has at least one nozzle (6) as the outlet.

34. Microstructured nozzle according to claims 1 to 31, **characterised by**
- a base plate (1) made of silicon and a cover plate made of silicon, attached by direct bonding.

35. Microstructured nozzle according to claims 1 to 34, **characterised in that** the spacings between the built-in elements, each of which forms a throughflow channel for the liquid passing through, are such that the resulting cross sectional area perpendicular to the direction of flow which is effectively permeable to the liquid is greater than the corresponding effective cross sectional area of the throughflow channels formed by the structures of the main filter.

36. Atomiser for inhalation therapy which comprises a microstructured nozzle according to one of the preceding claims 1 to 35.

37. Process for producing a nozzle according to one of claims 1 to 35, wherein in one step microstructures in the form of the filter structures, secondary structures, nozzle inlet and nozzle outlet are etched into one side of a silicon wafer for a large number of nozzles, in a subsequent step a glass plate is firmly attached to this side of the silicon wafer, in an independent step the silicon wafer is placed on an adhesive film and in a final step the individual nozzles are produced from the assembly comprising the silicon wafer and glass plate with the adhesive film on the underside of the silicon wafer, starting from the glass plate side, using a diamond saw.

## Revendications

1. Ajutage microstructuré comportant un filtre, une entrée pour du liquide non filtré et une sortie pour du liquide filtré, l'ajutage comprenant :
une plaque de fond (1) essentiellement plate et une plaque de recouvrement pouvant être fixée dessus ;
un filtre principal réalisé comme une structure primaire avec plusieurs saillies (7) disposées les unes à côté des autres en rangées (3), comme des composants intégraux de la plaque de fond (1) et qui sont éloignées respectivement de celle-ci, les saillies (7) étant espacées les unes des autres par des canaux (8) qui forment, de l'entrée à la sortie, une voie fluidique au travers de l'ajutage, la plaque de recouvrement lorsqu'elle est fixée sur la plaque de fond, recouvrant les saillies (7) et les canaux (8) ;
et
un espace collecteur de filtrat (50a) disposé dans le sens d'écouleznent derrière le filtre principal,
**caractérisé en ce que**
dans l'espace collecteur de filtrat (50a) est réalisée une structure secondaire (50) qui comporte une pluralité d'éléments de montage (51) appliqués sur la plaque de fond (1) et/ou la plaque de recouvrement, lesquels présentent un diamètre compris entre 0,005 et 0,02 mm.

2. Ajutage microstructuré comportant un filtre, une entrée pour du liquide non filtré et une sortie pour du liquide filtré, l'ajutage comprenant :
une plaque de fond (1) essentiellement plate et une plaque de recouvrement pouvant être fixée dessus ;
un filtre principal réalisé comme une structure primaire avec plusieurs saillies (7) disposées les unes à côté des autres en rangées (3), comme des composants intégraux de la plaque de fond (1) et qui sont éloignées respectivement de celle-ci et qui sont disposées dans une configuration en zigzag, les saillies (7) étant espacées les unes des autres par des canaux (8) qui forment une voie fluidique au travers de l'ajutage de l'entrée à la sortie, la plaque de recouvrement, lorsqu'elle est fixée sur la plaque de fond, recouvrant les saillies (7) et les canaux (8) ; et
un espace collecteur de filtrat (50a) disposé dans le sens d'ëcouleznent derrière le filtre principal,
**caractérisé en ce que**
dans l'espace collecteur de filtrat (50a) est réalisée une structure secondaire (50) qui comporte une pluralité d'éléments de montage (51) appliqués sur la plaque de fond (1) et/ou la plaque de recouvrement, lesquels peuvent être réalisés dans le sens d'écoulement devant et de manière desserrée derrière la configuration en zigzag.

3. Ajutage microstructuré selon la revendication 2, **caractérisé en ce que** les éléments de montage (51) présentent un diamètre compris entre 0,005 mm et 0,02 mm.

4. Ajutage microstructuré selon l'une quelconque des revendications 1 à 3, **caractérisé en ce que** les éléments de montage (51) présentent une paroi périphérique cylindrique.

5. Ajutage microstructuré selon l'une quelconque des revendications 1 à 4, **caractérisé en ce que** les éléments de montage (51) présentent entre eux une distance comprise entre 0,005 et 0,02 mm.

6. Ajutage microstructuré selon l'une quelconque des revendications 1 à 5, **caractérisé en ce que** les éléments de montage (51) présentent un diamètre de 0,01 mm.

7. Ajutage microstructuré selon l'une quelconque des revendications 1 à 6, **caractérisé en ce que** les éléments de montage (51) présentent une paroi périphérique concave.

8. Ajutage microstructuré selon l'une quelconque des revendications 1 à 6, **caractérisé en ce que** les éléments de montage (51) présentent une paroi périphérique convexe.

9. Ajutage microstructuré selon l'une quelconque des revendications 1 à 8, **caractérisé en ce que** les éléments de montage s'étendent comme des piliers de la plaque de fond à la plaque de recouvrement et font partie intégrante de la plaque de recouvrement.

10. Ajutage microstructuré selon l'une quelconque des revendications 1 à 9, **caractérisé en ce que** les saillies (7) sont disposées les unes à côté des autres sur toute la largeur du filtre.

11. Ajutage microstructuré selon l'une quelconque des revendications 2 à 10, **caractérisé en ce que** la structure secondaire (50) est réalisée du côté de la sortie de la configuration en zigzag jusqu'à la ligne médiane (52).

12. Ajutage microstructuré selon l'une quelconque des revendications 2 à 10, **caractérisé en ce que** la structure secondaire (50) est réalisée du côté de la sortie de la configuration en zigzag jusque dans les pointes s'élevant en direction de l'entrée.

13. Ajutage microstructuré selon l'une quelconque des revendications 1 à 12, **caractérisé par**
- une distance entre la plaque de fond dans les environs des saillies et la plaque de recouvrement dans une rangée (3) de saillies (7) qui est à peu près aussi grande que la largeur des canaux (8) du côté des saillies (7), duquel côté le liquide entre dans la rangée de canaux (8).

14. Ajutage microstructuré selon l'une quelconque des revendications 1 à 13, **caractérisé par**
- une pluralité de saillies (7) disposées les unes à côté des autres en rangées (3) qui dépassent d'une plaque de fond (1) et qui font partie intégrante de la plaque de fond,
- plusieurs rangées (3) de saillies étant disposées en cascade et
- la section transversale des canaux (8) se réduisant de rangée en rangée perpendiculairement au sens d'écoulement du liquide, vu dans le sens d'écoulement et
- les saillies (3) disposées plus près sur le côté d'entrée du filtre étant plus grandes ou leur nombre étant plus élevé de sorte que leurs distances deviennent plus petites que les saillies (3) disposées plus sur le côté de sortie du filtre et
- la distance entre la plaque de fond et la plaque de recouvrement dans les environs de chaque rangée de saillies (7) disposée en cascade étant à peu près aussi grande que la largeur des canaux du côté des saillies (7), duquel entre le liquide dans la rangée de canaux, et
- une fente d'entrée oblongue (5) pour le liquide non filtré qui s'étend sur approximativement toute la largeur de filtre et qui est à peu près aussi haute que les saillies (7) dépassant de la plaque de fond du côté d'entrée du filtre, et
- une fente de sortie oblongue pour le liquide filtré qui s'étend sur approximativement toute la largeur de filtre et qui est à peu près aussi haute que les saillies (7) dépassant de la plaque de fond du côté de sortie du filtre.

15. Ajutage microstructuré selon les revendications 1 à 14, **caractérisé par**
- une plaque de fond (1) plate et une plaque de recouvrement plate.

16. Ajutage microstructuré selon les revendications 1 à 15, **caractérisé en ce que** toutes les structures du filtre sont réalisées exclusivement sur la plaque de fond (1).

17. Ajutage microstructuré selon les revendications 1 à 16, **caractérisé par**
- une distance entre la plaque de fond (1) plate dans les environs des saillies (7) et la plaque de recouvrement plate dans une rangée (3) de saillies (7) entre la moitié et le double de la largeur de canal du côté des saillies (7), duquel entre le liquide dans la rangée de canaux (8).

18. Ajutage microstructuré selon les revendications 1 à 17, **caractérisé par** plusieurs rangées de saillies les unes à côté des autres, les côtés tournés les uns vers les autres de deux rangées contigües de saillies (7) délimitant un espace communicant, dans lequel le liquide s'écoule de tous les canaux entre les saillies d'une première rangée et duquel s'écoule le liquide dans tous les canaux entre les saillies de la rangée suivante dans le sens d'écoulement.

19. Ajutage microstructuré selon les revendications 1 à 18, **caractérisé en ce que**
- l'espace collecteur (50a) présente une section transversale oblongue entre la fente d'entrée (5) et la première rangée de saillies (4), dans laquelle le liquide non filtré est introduit et de laquelle s'écoule le liquide dans tous les canaux entre les saillies de la première rangée, et **en ce que**
- l'espace collecteur (50a) présente une section transversale oblongue entre la dernière rangée de saillies et la fente de sortie, dans laquelle s'écoule le liquide de tous les canaux de la dernière rangée, et duquel le liquide filtré est dérivé.

20. Ajutage microstructuré selon les revendications 1 à 19, **caractérisé par**
- des saillies en forme de traverses (11, 12, 13, 14, 15) qui sont droites ou courbées, vu dans le sens d'écoulement ou
- des saillies en forme de colonnes (16, 17, 18, 19).

21. Ajutage microstructuré selon les revendications 1 à 20, **caractérisé par**
- des canaux (8), dont la longueur, à section transversale constante, est au moins deux fois plus grande que leur hauteur du côté d'entrée du liquide.

22. Ajutage microstructuré selon les revendications 1 à 21, **caractérisé par**
- des canaux (8) s'étendant entre des traverses avec une section transversale approximativement carrée qui est constante sur la longueur de canal, avec une longueur de 5 à 50 micromètres, avec une hauteur de 2,5 à 25 micromètres et une largeur de 2,5 à 25 micromètres.

23. Ajutage microstructuré selon les revendications 1 à 22, **caractérisé par**
- des canaux, dont la section transversale est en forme de tonneau ou de trapèze, le côté le plus long du trapèze étant formé de préférence par la plaque de recouvrement.

24. Ajutage microstructuré selon les revendications 1 à 23, **caractérisé par**
- des canaux (8) avec une section transversale approximativement carrée du côté d'entrée du liquide et une section transversale s'élargissant vers le côté de sortie du liquide.

25. Ajutage microstructuré selon les revendications 1 à 24, **caractérisé par**
- une distance entre les rangées de saillies qui est de préférence deux fois plus grande que la largeur de canal du côté d'entrée.

26. Ajutage microstructuré selon les revendications 1, 4 à 10 ou 13 à 25, **caractérisé par**
- des saillies qui sont inclinées les unes contre les autres
- dans des rangées (31) s'étendant parallèlement les unes aux autres ou
- dans des rangées (33) s'étendant en forme de méandre d'un angle alpha de 2 à 25 degrés.

27. Ajutage microstructuré selon les revendications 2 à 25, **caractérisé par** des saillies qui sont inclinées les unes contre les autres d'un angle alpha de 2 à 25 degrés.

28. Ajutage microstructuré selon les revendications 1 à 27, **caractérisé par**
- une distance constante entre la plaque de fond (1) plate dans les environs des saillies (7) et la plaque de recouvrement plate dans une rangée (3) de saillies.

29. Ajutage microstructuré selon les revendications 1 à 27, **caractérisé par** une distance se rétrécissant dans le sens d'écoulement entre la plaque de fond (1) et la plaque de recouvrement.

30. Ajutage microstructuré selon les revendications 1 à 29, **caractérisé par**
- une distance entre la plaque de fond (1) plate dans les environs des saillies (7) et la plaque de recouvrement plate dans une rangée de saillies qui augmente de la zone de l'extrémité de rangée qui se trouve à proximité du côté d'entrée du filtre, en direction de la zone de l'extrémité de rangée qui se trouve à proximité du côté de sortie du filtre.

31. Ajutage microstructuré selon les revendications 1 à 30, **caractérisé par**
- une plaque de fond (1) qui a été structurée par corrosion par voie humide ou sèche isotrope ou anisotrope ou une combinaison de ces procédés, de préférence par corrosion par voie sèche anisotrope.

32. Ajutage microstructuré selon les revendications 1 à 31, **caractérisé par**
- une plaque de fond (1) en silicium et une plaque de recouvrement en verre qui est assemblée par métallisation anodique.

33. Ajutage microstructuré selon les revendications 1 à 30, **caractérisé en ce que** l'espace collecteur de filtrat (50a) se termine en cône dans le sens d'écoulement et présente comme sortie au moins un ajutage (6).

34. Ajutage microstructuré selon les revendications 1 à 31, **caractérisé par**
- une plaque de fond (1) en silicium et une plaque de recouvrement en silicium qui est assemblée par métallisation directe.

35. Ajutage microstructuré selon les revendications 1 à 34, **caractérisé en ce que** les distances entres les éléments de montage qui forment respectivement un canal de passage pour le liquide qui s'écoule sont telles que l'aire de la section effective perméable au liquide, résultant perpendiculairement au sens d'écoulement est plus grande que l'aire de la section effective correspondante des canaux de passage qui sont formés par les structures du filtre principal.

36. Pulvérisateur pour le traitement par inhalation, qui comporte un ajutage microstructuré selon l'une quelconque des revendications précédentes 1 à 35.

37. Procédé pour fabriquer un ajutage selon l'une quelconque des revendications 1 à 35, dans lequel
des microstructures sous la forme de structures de filtre, de structures secondaires, d'entrée d'ajutage et de sortie d'ajutage sont corrodées pour un grand nombre d'ajutages dans une étape d'un côté d'une tranche de silicium,
dans une étape suivante une plaque de verre, est fermement fixée de ce côté de la tranche de silicium,
dans une étape indépendante de celle-ci, la tranche de silicium est appliquée sur un film adhésif, et
dans une étape finale, les ajutages individuels sont façonnés dans l'unité structurelle composée de la tranche de silicium et de la plaque de verre avec le film adhésif sur le côté inférieur de la tranche de silicium venant du côté de la plaque de verre à l'aide d'une scie diamant.
